# EUROPEAN PATENT APPLICATION

(11) **EP 1 013 768 A1**
(43) Date of publication of application: **28.06.2000**
(21) Application number: 98204315.0
(22) Date of filing: 18.12.1998
(51) Int. Cl.: C12N 15/55, C12N 9/14, C12N 15/70, C12N 1/21, C12P 41/00

(54) **Mutant epoxide hydrolases**

(71) Applicant: DSM N.V., 6411 TE Heerlen (NL)
(72) Inventor: Rink, Rick, 9717 AG Groningen (NL); Lutjes Spelberg, Jeffrey Harald, 9743 PA Groningen (NL); Nardini, Marco, 9717 CE Groningen (NL); Dijkstra, Bauke Wiepke, 9717 GA Groningen (NL); Kellogg, Richard Morrisen, 9752 VW Haren (NL); Janssen, Dirk Barend, 9301 KP Roden (NL)

(57) **Abstract**

Mutant epoxide hydrolases having an amino acid substitution of a tyrosine residue in the cap domain of the wild type epoxide hydrolase, in particular mutant epoxide hydrolases having an amino acid substitution of the tyrosine residue corresponding to Tyr¹⁵² or Tyr²¹⁵ in an epoxide hydrolase with an aminoacid sequence according to SEQ ID No. 1. Preferably the Tyr is substituted by Phe. The invention also relates to nucleic acid sequences encoding such mutant epoxide hydrolases expression constructs wherein such nucleic acid sequence according is operably linked to a regulatory region capable of directing the expression of the mutant epoxide hydrolase in a suitable expression host, vectors capable of transforming a host cell characterized in that the vector containing such expression construct, transformed host cells transformed with such a vector, processes for the preparation of such mutant epoxide hydrolases and processes for the preparation of optically active epoxides and/or diols using such mutant epoxide hydrolases.

SEQUENCE LISTING

<110> DSM N.V.

<120> Mutant Epoxide  Hydrolases

<130> 9815ep

<140> 98204315.0
<141> 1998-12-18

<160> 1

<170> PatentIn Ver. 2.1

<210> 1
<211> 294
<212> PRT
<213> Agrobacterium radiobacter

<400> 1
Met Thr Ile Arg Arg Pro Glu Asp Phe Lys His Tyr Glu Val Gln Leu
  1               5                  10                  15

Pro Asp Val Lys Ile His Tyr Val Arg Glu Gly Ala Gly Pro Thr Leu
             20                  25                  30

Leu Leu Leu His Gly Trp Pro Gly Phe Trp Trp Glu Trp Ser Lys Val
         35                  40                  45

Ile Gly Pro Leu Ala Glu His Tyr Asp Val Ile Val Pro Asp Leu Arg
     50                  55                  60

Gly Phe Gly Asp Ser Glu Lys Pro Asp Leu Asn Asp Leu Ser Lys Tyr
 65                  70                  75                  80

Ser Leu Asp Lys Ala Ala Asp Asp Gln Ala Ala Leu Leu Asp Ala Leu
                 85                  90                  95

Gly Ile Glu Lys Ala Tyr Val Val Gly His Asp Phe Ala Ala Ile Val
            100                 105                 110

Leu His Lys Phe Ile Arg Lys Tyr Ser Asp Arg Val Ile Lys Ala Ala
        115                 120                 125

Ile Phe Asp Pro Ile Gln Pro Asp Phe Gly Pro Val Tyr Phe Gly Leu
    130                 135                 140

Gly His Val His Glu Ser Trp Tyr Ser Gln Phe His Gln Leu Asp Met
145                 150                 155                 160

Ala Val Glu Val Val Gly Ser Ser Arg Glu Val Cys Lys Lys Tyr Phe
                165                 170                 175

Lys His Phe Phe Asp His Trp Ser Tyr Arg Asp Glu Leu Leu Thr Glu
            180                 185                 190

Glu Glu Leu Glu Val His Val Asp Asn Cys Met Lys Pro Asp Asn Ile
        195                 200                 205

His Gly Gly Phe Asn Tyr Tyr Arg Ala Asn Ile Arg Pro Asp Ala Ala
    210                 215                 220

Leu Trp Thr Asp Leu Asp His Thr Met Ser Asp Leu Pro Val Thr Met
225                 230                 235                 240

Ile Trp Gly Leu Gly Asp Thr Cys Val Pro Tyr Ala Pro Leu Ile Glu
                245                 250                 255

Phe Val Pro Lys Tyr Tyr Ser Asn Tyr Thr Met Glu Thr Ile Glu Asp
            260                 265                 270

Cys Gly His Phe Leu Met Val Glu Lys Pro Glu Ile Ala Ile Asp Arg
        275                 280                 285

Ile Lys Thr Ala Phe Arg
    290

## Description

The invention relates to epoxide hydrolases having an amino acid substitution of a tyrosine residue in the cap domain of an epoxide hydrolase, nucleic acid sequences encoding such mutant epoxide hydrolases, vectors comprising such nucleic acid sequences, micro-organism host cells transformed with such vectors and to a process for the enantioselective enzymatic hydrolysis of chiral epoxides to obtain optically active epoxides and diols, using such epoxide hydrolases.

Both optically enriched epoxides and diols for instance having an enantiomeric excess (ee) of more than 80%, especially optically pure epoxides and diols having an enantiomeric excess of more than 90% preferably more than 95%, in particular more than 98%, are important building blocks in organic synthesis. Despite progress in their preparation, no general and efficient methods are currently available. A promising method is the kinetic resolution of readily available racemic epoxides leading to epoxides of high optical purity. Epoxide hydrolases that perform this task are found in a whole range of organisms and increasing numbers have been identified.

Amino acid sequence similarity studies between soluble- and microsomal epoxide hydrolases of mammalian origin and haloalkane dehalogenase from *Xanthobacter autotrophicus* have revealed low but significant homology. It has therefore been suggested that, like haloalkane dehalogenase, also the mammalian epoxide hydrolases, belong to the α/β-hydrolase fold family of enzymes (e.g. M. Arand, D.F. Grant, J.F. family of enzymes (e.g. M. Arand, D.F. Grant, J.F. Beetham, T. Friedberg, F. Oesch and B.D. Hammock, (1994) FEBS-letters, 338, 251-256). This α/β hydrolase fold is a conserved structural topology with a main domain that consists of a central β-sheet that is alternated with α-helices that cover both sides (D.L. Ollis, E. Cheah, M. Cygler, B.W. Dijkstra, F. Frolow, S.M. Franken, M. Harel, S.J. Remington, I. Silman, J. Schrag, J.L. Sussman, K.H.G. Verschueren and A. Goldman. Protein Eng. 5:197 (1992)).

Also bacterial epoxide hydrolases belong to the α/β hydrolase fold enzymes as has been shown for the epoxide hydrolase from *Agrobacterium radiobacter* AD1 by Rink et al. (1997) from amino acid sequence similarity with haloalkane dehalogenase and bromoperoxidase A2 and secondary structure predictions.

The α/β hydrolase fold enzymes in general and epoxide hydrolases in particular consist of one strand of amino acids. This strand can be divided in 3 parts of which the N-terminus part and the C-terminus part together form the core domain. The core domain is characterized by high similarity and high content of structural features (e.g. α-helices and β-sheets). Furthermore the core domain contains the active site residues which are highly conserved in α/β hydrolase fold enzymes in general and epoxide hydrolases in particular. The third part of the strand that separates the two parts of the core domain is known as the "cap" domain. This "cap" domain constitutes a second domain in epoxide hydrolases and covers the active site in the core domain like a cap.

The parts of the amino acid strand forming the core- and the cap domain of an epoxide hydrolase can in general be identified by:
* Aligning its amino acid sequence with one or more known α/β-hydrolase fold enzymes for which not only the amino acid sequence but also the 3-dimensional structure is known, or by
* Predicting the secondary structure elements of the epoxide hydrolase and comparing these with the secondary structure elements of the 3-dimensional structure of the α/β hydrolase fold enzyme.
Examples of suitable α/β hydrolase fold enzymes are haloalkane dehalogenase, bromoperoxidase A2 and others given by Rink et al (1997).
The (multiple) alignment of amino acid sequences can be carried out using computer programs known in the art (e.g. Clustal-W vl.6; J.D. Thompson, D.G. Higgins and T.J. Gibson, Nucleic Acids Res. 22, 4673-4680). Methods for secondary structure predictions are also known in the art and are widely and freely available from the World Wide Web. Examples are SopM (C. Geourjon and G. Deleage, Protein Eng. 7. 157-164, (1994)), ssp (V.V. Solovyev and A.A. Salamov, Molek.Biol. 25, 810-824, (1991)), Sspred (P.K. Mehta, J. Heringa and P. Argos, Protein Sci. 4, 2517-2525, (1995)) and Predict-Protein (B. Rost and C. Sander, Proteins, 19, 55-72, (1994)).

By applying the methods described above it was found that the cap domain of epoxide hydrolases is located between beta-strands 6 and 7 and comprises amino acid residues that correspond to residues ∼130 - ∼230 of the epoxide hydrolase of *Agrobacterium radiobacter AD1* (the complete DNA and amino acid sequence, SEQ ID No. 1, of the epoxide hydrolase gene from Agrobacterium radiobacter deposited at the Centraal bureau voor Schimmelcultures under number is given in fig. 1 of WO-A-9853081 and in R. Rink, M. Fennema, M. Smids, U. Dehmel and D.B. Janssen, J. Biol, Chem. 272, 14650-14657 (1997)) and haloalkane dehalogenase of *Xanthobacter autotrophicus*.

The mechanism of epoxide hydrolases has already been discussed in the literature and involves two steps (analogous to haloalkane dehalogenase - K.H.G. Verschueren, F. Seljeé, H.J. Rozenboom, K.H. Kalk and B.W. Dijkstra, Nature 363, 693-698 (1993)). In the first reaction step an ester bond is formed between enzyme and substrate by attack of the nucleophile Asp107 on one of the carbon atoms of the epoxide ring of the substrate; in the second step, this ester bond is hydrolyzed by a water molecule activated by the His275/Asp246 pair (Rink et al. (1997)). In the first step, the oxyanion formed during the opening of the epoxide ring should be protonated by a proton donating group of the cap domain. In the literature, one of the three lysines in the cap domain, Lys173, Lys174 or Lys177, is proposed to be involved in the protonation of the leaving product (e.g. Rink et al. (1997)).

We have surprisingly found that tyrosine residues in the cap domain of epoxide hydrolases are involved in the catalytic mechanism and determine the enantioselectivity of epoxide hydrolases and the mutant epoxide hydrolases having an amino acid substitution of such tyrosine residue show improved properties. Moreover, as illustrated in Tables 1 and 2, it has appeared by sequence alignment of known epoxide hydrolases according to the methods described above, that said tyrosines, in particular those corresponding to position 215 (Table 1) and 152 (Table 2) of the epoxide hydrolase of *Agrobacterium radiobacter AL1* as described in WO-A-98/53081 and in Rink et al. (1997) (with an aminoacid sequence according to SEQ ID No.1 as given in these publications), are conserved among the various epoxide hydrolases known today. Therefore, the involvement of these cap domain tyrosines in the catalytic mechanism and in the enantioselectivity of the enzyme, should be regarded as a universal property.

**Table 1**

| **soluble epoxide hydrolases** | |
|---|---|
| Agr | NIHGGFNYYRANIRPDAALWTDLDHTMSDLPV 238 |
| Cor | SIRSGCQWYATGLREDTENLAKATDKLTIPVI 232 |
| Tha | GFSGPVNYYRNFNRNNELLGPWVGSKIQVPTK 258 |
| Soy | GFTGPLNYYRNFNLNWELTAPWTGGQIKVPVK 278 |
| Pot | GFTGAVNYYRALPINWELTAPWTGAQVKVPTK 258 |
| Hum | GFRGPLNWYRNMERNWKWACKSLGRKILIPAL 488 |
| Mou | GFRGPLNWYRNTERNWKWSCKGLGRKILVPAL 487 |
| Rat | GFRGPLNWYRNTERNWKWSCKALGRKILVPAL 488 |

| **microsomal epoxide hydrolases** | |
|---|---|
| Hum | TIISSQRFYKENLGQGWMTQKHERMKVYVPTG 397 |
| Rab | SIVSSQRYYKENLGQGFMAHKHERLKVHVPTG 397 |
| Rat | TIVSSQRYYKENLGQGIMVHKHEGMKVFVPTG 397 |
| Mou | TIVSSQRFYKENLGQGVMVHRHEGMKVFVPTG 397 |
| Pig | TITSSQRFYKENLGQGVMANKHEAIKVHVPTG 396 |
| Hor | CIVTSTRLYAEGFS-WPEVLIVHRIPSMVPTA 394 |
| Cab | AITPAMRLYAENFNKRTVEMKLDEIPTPVPTW 396 |
| Abbreviations are written in full: (Agr)obacterium radiobacter AD1; (Cor)ynebacterium sp. C10; (Tha)lecress (= Arabidopsis thalania); (Soy)bean; (Pot)ato; (Hum)an; (Mou)se; (Rab)bit; (Hor)nworm; (Cab)bagelooper. | |

**Table 2**

| | |
|---|---|
| Agrobacterium | QPDFGPVYFGLGHVHESWYSQFHQLDMAVEV 164 |
| Arabidopsis | DPSVKPVDRMRAFYGDDYYICRFQEFGDVEA 164 |
| Soybean | DPNIRTVDGMRALYGDDYYVCRFQKPGEMEA 175 |
| Potato | NPKMNVVEGLKAIYGEDHYISRFQVPGEIEA 164 |
| Human | NPNMSPLESIKANPVFD-YQLYFQEPGVAEA 392 |
| Mouse | DPDVSPMKVIRSIPVFN-YQLYFQEPGVAEA 390 |
| Rat | NPEVSPMEVIRSIPVFN-YQLYFQEPGVAEA 391 |

In one aspect of the invention epoxide hydrolase mutant enzymes are provided with at least a mutation in one or more of the positions corresponding to 141, 147, 152, 175, 185, 197, 198, 214 or 215 in the epoxide hydrolase of *Agrobacterium radiobacter AD1*.

In another aspect of the invention, epoxide hydrolase mutants are provided that possess a tyrosine in the wild type enzyme in any of the positions corresponding to 141, 147, 152, 175, 185, 197, 198, 214 or 215 in the epoxide hydrolase of *Agrobacterium radiobacter AD1* and in which said tyrosine has been replaced by another (proteinogenic) amino acid.

According to a further aspect of the invention, epoxide mutant enzymes are provided with substituted tyrosines at positions 141, 152, 175, 185, 214 and 215 of the epoxide hydrolase from *Agrobacterium radiobacter AD1*.

In another aspect of the invention, epoxide mutant enzymes are provided with a substituted tyrosine in the cap domain. For the example of the soluble epoxide hydrolase from potato these are the positions corresponding to the positions 149, 154, 183, 190, 218, 219, 234 and 235 in Agrobacterium radiobacter AD1.

According to a further aspect of the invention, a mutant epoxide hydrolase from *Agrobacterium radiobacter AD1* is provided in which tyrosine 152 or tyrosine 215 is substituted by phenylalanine and which mutant enzyme is characterized by an enhanced enantioselectivity for a variety of epoxide substrates.

According to an aspect of the invention there is provided a nucleic acid sequence encoding a mutant epoxide hydrolase of the invention.

According to a further aspect of the invention there is provided a DNA construct in which a DNA sequence encoding the mutant epoxide hydrolase of the invention is operably linked to a regulatory region capable of directing the expression of the DNA sequence encoding the mutant epoxide hydrolase in a suitable host cell. Regulatory regions are herein understood to include promoters and upstream regulatory sequences necessary for initiation and regulation of transcription and translation; and optionally transcription termination sequences and, in case of eukaryotic host cells poly-adenylation sequences. Optionally the DNA construct may also contain DNA sequences encoding signal sequences for directing secretion of the nascent polypeptide.

Another aspect of the invention provides host cells transformed with a vector comprising a DNA construct for the expression of a DNA sequence encoding a mutant epoxide hydrolase of the invention. A variety of suitable host cells for the production of a mutant epoxide hydrolase of the invention are available to the skilled person. Preferred host cells are of microbial origin, for instance bacteria, yeast and filamentous fungi. Preferred bacterial host cells include *Escherichia coli*, *Agrobacterium* species*, Bacillus* species and *Streptomyces* species. Preferred yeast host cells are selected from the group consisting of the genera *Saccharomyces*, *Kluyveromyces*, *Hansenula*, *Pichia*, *Yarrowia*, and *Candida*. Preferred filamentous fungal host cells are selected from the group consisting of the genera *Aspergillus*, *Trichoderma*, and *Fusarium*.

According to one aspect of the invention there is provided a process for the preparation of mutant epoxide hydrolase of the invention, wherein the process comprises culturing a host cell transformed with a DNA construct capable of expressing a mutant epoxide hydrolase of the invention, under conditions conducive to the expression of the hydrolase. Preferably the process further comprises recovery of the mutant epoxide hydrolase of the invention.

The epoxide hydrolase to be mutated is preferably obtainable from bacteria, for instance a Rhodococcus, a Bacillus, a Pseudomonas, a Nocardia, a Mycobacterium, a Corynebacterium or an Agrobacterium species; from fungi, for instance a Beauvaria or an Aspergillus species; from plants for instance thale cress, soybean or potato; or microsomal epoxide hydrolases, for instance from human, rabbit, rat, mouse, pig, hornworm or cabbagelooper.
According to a preferred aspect of the invention the epoxide hydrolase to be mutated is obtainable from a fungus from the Aspergillus niger group, including all (black) Aspergilli than can be found in the Aspergillus niger group as defined by Raper and Fennell (1965, In: The Genus Aspergillus, The Williams & Wilkins Company, Baltimore, pp 293-344).

The epoxides that can be used as a substrate can be chosen from mixtures, e.g. racemic mixtures, of the enantiomers of a wide range of compounds, e.g. aliphatic epoxides, for instance epoxyalkanes; (hetero)aromatic epoxides, for instance (o-, m- or p- halo- or nitro-)styrene oxides; glycidic acids or esters, for instance (p-methoxy-)phenylglycidic acid or esters thereof; ethers, for instance phenylglycidylether; or epoxides wherein the 2 C-atoms in the epoxide-ring are part of a second ring, for instance indene-oxide; optionally substituted 2,3- or 3,4-chromenepoxides.

The invention is further illustrated by the following examples without limiting the scope of the invention.

### Example I

### Gene cloning and site directed mutagenesis.

The gene coding for the epoxide hydrolase gene from Agrobacterium radiobacter AD1 (deposit number CBS 750.97) was cloned and sequenced as described by Rink et al. (J. Biol. Chemistry, 272, 14650-14657 (1997)).

Site directed mutagenesis was done as described by Kunkel (Proc. Natl. Acad. Sci. U.S.A., 82, 488-492 (1985)) with uracil-containing single stranded plasmid pEH20 as template (Rink et al.). Tyr²¹⁵ was mutated to Phe with the primer 5'-CAACTACTTCCGTGACCAAAC-3' (mutated codon underlined). In order to confirm the mutation, the whole gene was sequenced as described in Rink et al.

### Example II

### Expression and purification of epoxide hydrolase.

Both wild type and mutant epoxide hydrolase were expressed in Escherichia coli BL21 (DE3). The transformation of E. coli, expression of the gene and culturing of transformed cells has been described in detail by Rink et al.

The purification of the enzyme involved collecting and disrupting the cells by sonication followed by several sequential column chromatographies as described by Rink et al. The purified enzymes (wild type and mutant) were stored at 4°C, while 10% (v/v) glycerol was used for its stabilising effect on the enzyme upon storage.

### Example III

### Stereoselective hydrolysis.

A screw-cap bottle containing 30 ml Tris buffer¹ (50 mM pH9) was incubated in a shaking waterbath at 30°C. Styrene oxides (SO) as indicated in table 3 were added to a final concentration of 5 mM.
The reaction was started by the addition of the a purified enzyme solution of the Tyr215Phe mutant of the epoxide hydrolase from Agrobacterium radiobacter AD1. The kinetic resolution was monitored by periodically taking 1 ml samples (with a 1 ml syringe) from the closed screw-cap bottle. The samples were extracted with diethylether containing 20 mM mesitylene as an internal standard. Prior to analysis by chiral GLC the water was removed from the sample by passing it through a short column containing MgSO₄. The enantiomeric excess (%ee) and the yield of the epoxides was determined with a Hewlett-Packard 5890 gas chromatograph equipped with a FID-detector, using a Chiraldex G-TA capillary column, of 50 m lenght and 0.25 mm inside diameter. The data from the chiral GLC are as follows:
styrene oxide, tᵣ(S)=12.4 min and tᵣ(R)=14.8 min at 110°C;
meta-chlorostyrene oxide, tᵣ(S)=20.2 min and tᵣ(R)= 31.1 min at 120°C;
para-chlorostyrene oxide, tᵣ(S)=40.7 min and tᵣ(R)= 43.0 min at 100°C.

¹ In case of meta-chlorostyrene oxide and para-chlorostyrene oxide the buffer also contained 10% DMSO

The corresponding E-values are given in table 3.

### Comparative Experiment

Example III was repeated using the corresponding wild type epoxide hydrolase for Agrobacterium radiobacter AD₁.
The corresponding E-values are given in table 3.

**Table 3**

| | E-value | |
|---|---|---|
| Substrate | Wild type | Tyr215Phe |
| P-NO₂-SO | 100 | > 200 |
| SO | 16 | 30 |
| M-Cl-SO | 6.5 | 13 |
| P-Cl-SO | 32 | 130 |

### Example IV

Example III was repeated using racemic styrene oxide as a substrate and using the Tyr152Phe mutant as an enzyme. The E-value now was 40 or higher.

## Claims

1. A mutant epoxide hydrolase having an amino acid substitution of a tyrosine residue in the cap domain of the wild type epoxide hydrolase.

2. A mutant epoxide hydrolase according to claim 1 having an amino acid substitution of the tyrosine residue corresponding to Tyr¹⁵² or Tyr²¹⁵ in an epoxide hydrolase with an aminoacid sequence according to SEQ ID No. 1.

3. A mutant epoxide hydrolase according to claim 1 or 2, wherein Tyr is substituted by Phe.

4. A mutant epoxide hydrolase according to any one of claim 1-3, wherein the epoxide hydrolase is obtainable from Agrobacterium, Corynebacterium, Nocardia, Mycobacterium, Bacillus, Rhodococcus, Pseudomonas, Aspergillus or Beauvaria.

5. A nucleic acid sequence encoding a mutant epoxide hydrolase as defined in anyone of claims 1-4.

6. An expression construct characterized in that the nucleic acid sequence according to claim 5 is operably linked to a regulatory region capable of directing the expression of the mutant epoxide hydrolase in a suitable expression host.

7. A vector capable of transforming a host cell characterized in that the vector contains an expression construct according to claim 6.

8. A transformed host cell characterized in that it is transformed with a vector of claim 7.

9. The transformed host cell of claim 8 which is selected from the group consisting of bacteria, yeast of fungi.

10. The transformed host cell of claim 9 which is the genus Agrobacterium, Aspergillus, Trichoderma, Fusarium, Bacillus, Candida, Escherichia, Hansenula, Kluyveromyces, Pichia, Saccharomyces Streptomyces or Yarrowia.

11. A process for the preparation of a mutant epoxide hydrolase according to anyone of claims 1-3, comprising culturing a transformed host cell according to anyone of claims 8-10 under conditions conducive to the expression of the mutant epoxide hydrolase

12. A process according to claim 11 wherein the mutant epoxide hydrolase is recovered.

13. A process for the preparation of an optically active diol and/or epoxide wherein a mixture of the enantiomers of a chiral epoxide is subjected to an enzymatic hydrolysis in the presence of an epoxide hydrolase according to anyone of claims 1-4.

14. A mutant epoxide hydrolases and processes for the preparation of optically active diols and/or epoxides as described and elucidated by the examples.
